Europäisches Patentamt

European Patent Office

Office européen des brevets

(11) Veröffentlichungsnummer: **0 046 601**
**A2**

(12) # EUROPÄISCHE PATENTANMELDUNG

(21) Anmeldenummer: 81106645.5

(22) Anmeldetag: 26.08.81

(51) Int. Cl.³: **G 01 N 21/31**
**A 61 B 5/14**

(30) Priorität: 26.08.80 DE 3032150

(43) Veröffentlichungstag der Anmeldung:
03.03.82 Patentblatt 82-9

(84) Benannte Vertragsstaaten:
CH DE FR GB LI NL SE

(71) Anmelder: Hellige GmbH
Postfach 728 Heinrich-von-Stephan-Strasse 4
D-7800 Freiburg(DE)

(72) Erfinder: Schlüssel, Peter, Phys.-Ing. (grad.)
Goethestrasse 19
D-78000 Freiburg(DE)

(74) Vertreter: Ter Meer-Müller-Steinmeister Patentanwälte
Triftstrasse 4
D-8000 München 22(DE)

(54) Verfahren und Messeinrichtung zur kolorimetrischen Bestimmung der Konzentration eines chemischen Stoffs, insbesondere des Partialdrucks eines im Blut gelösten Gases.

(57) Dem Verfahren und der Vorrichtung zur kolorimetrischen Bestimmung der Konzentration eines im Blut gelösten Stoffes, insbesondere Gases liegt eine bichromatische Messung an einem den Stoff vorzugsweise auf transkutanem Wege aufnehmenden, langzeitstabilen opto-chemischen Wandler (6) zugrunde. Der Wandler (6) wird mit zwei diskreten Wellenlängenwerten $\lambda_0$ und $\lambda_{1,2}$ beaufschlagt, von denen $\lambda_0$ dem isosbestischen Wellenlängenwert, entspricht und $\lambda_{1,2}$ in einem Spektralbereich deutlicher Abhängigkeit des Extinktionsmoduls des Wandlers (6) liegt. Das Wandlermeßsignal wird elektronisch in ein Farbwertesignal $B(p)_{\lambda,T}$ umgesetzt, gegen vorgespeicherte Standard-Farbwertesignale verglichen und bestimmten ebenfalls vorgespeicherten Stoffkonzentratioswerten zugeordnet. Bei einer ersten Verfahrensvariante (Differentialverfahren mit Referenz) beruht das Meßprinzip auf der Abhängigkeit des spektralen Differentialquotienten 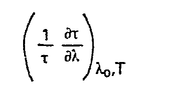 des Transmissionsgrades $\tau$ vom pH-Wert einer Farbstofflösung, während bei einer anderen Verfahrensvariante (Differentialverfahren ohne Referenz) die Abhängigkeit des spektralen Differentialquotienten

$$\left( \frac{1}{\tau} \frac{\partial \tau}{\partial \lambda} \right)_{\lambda_0, T}$$

des Transmissionsgrades $\tau$ vom pH-Wert einer Farbstofflösung im isosbestischen Punkt, also beim Wellenlängenwert $\lambda_0$ erfaßt wird.

./...

EP 0 046 601 A2

FIG. 5

FIG. 5a

PATENTANWÄLTE

# TER MEER-MÜLLER-STEINMEISTER

Beim Europäischen Patentamt zugelassene Vertreter — Professional Representatives before the European Patent Office
Mandataires agréés près l'Office européen des brevets

Dipl.-Chem. Dr. N. ter Meer
Dipl.-Ing. F. E. Müller
Triftstrasse 4,
D-8000 MÜNCHEN 22

Dipl.-Ing. H. Steinmeister
Artur-Ladebeck-Strasse 51
D-4800 BIELEFELD 1
Tel. (0521) 15 00 36 / 15 00 37

PW-P 435-EP
Mü/kü

26. August 1981

HELLIGE GMBH

Heinrich-von-Stephan-Str. 4

7800 Freiburg

---

Verfahren und Meßeinrichtung zur kolorimetrischen
Bestimmung der Konzentration eines chemischen Stoffs,
insbesondere des Partialdrucks eines im Blut gelösten
Gases

---

Priorität: 26. August 1980, Bundesrepublik Deutschland
P 30 32 150.7

## BESCHREIBUNG

Die Erfindung betrifft ein Verfahren und eine Meßeinrichtung zur kolorimetrischen Bestimmung der Konzentration
eines Stoffes, insbesondere des Partialdrucks eines im
Blut gelösten Gases. Die bekannte Gattung solcher Verfahren bzw. Meßeinrichtungen ist im Oberbegriff der Patentansprüche 1 bzw. 4 angegeben.

HELLIGE GMBH
PW-P 435-EP

- 2 -

Bekannte Verfahren zur kolorimetrischen Bestimmung der Konzentration von gelösten Stoffen im Blut, also etwa des Partialdrucks von Gasen wie Kohlendioxid und Sauerstoff oder der Wasserstoffinionenkonzentration, also des pH-Werts, unterscheiden nach der Anwendungsart zwischen transkutaner (US-PS 4 041 932, DE-OS 26 37 501) und intraversaler (DE-OS 28 51 138) Applikation der Meßwertaufnehmer oder Sonden, wobei die Messung sowohl mit Lichttransmission als auch durch Streulichtmessung erfolgen kann. Da das Meßsignal stark vom Strahlungsfluß des elektromagnetischen, in der Regel optischen Senders, der spektralen Empfindlichkeit des optischen Empfängers, der Temperatur und Konzentration des Farbstoffs usw. abhängt, ist in allen Fällen, die ohne Referenzsignal (Leerwert) arbeiten, selbst bei polychromatischen Verfahren (US-PS 4 041 932, Fig. 10) eine Kalibrierung des Meßgeräts für beispielsweise 0% und 100% des Meßwertebereichserforderlich.

Die meisten bekannten kolorimetrischen Verfahren etwa zur transkutanen Erfassung des Gasstoffwechsels an Mensch oder Tier arbeiten nach dem sogenannten optischen pH-Meßverfahren, bei dem ein quantitativ zu erfassendes gasförmiges Stoffwechselprodukt, bei der transkutanen Messung beispielsweise über eine selektiv permeable Membran, in eine Meßkammer eindiffundieren gelassen wird, die einen opto-chemischen Wandler, also insbesondere eine chemische Lösung - etwa Phenolrot - zur $CO_2$-Bestimmung enthält, deren optische Eigenschaften, insbesondere deren Transmissionsvermögen, sich für eine bestimmte Meßstrahlung in Abhängigkeit von der Menge des aufgenommenen Stoffwechselprodukts reversibel ändert. Die bekannten realisierten Geräte, die nach diesen Meßverfahren arbeiten, weisen jedoch verschiedene Mängel hinsichtlich der Meßbedingungen und Meßmethodik auf, die notwendigerweise sowohl zu zeit- als auch zu bedienungsintensiver Anwendung führen.

Der Erfindung liegt die Aufgabe zugrunde, ein Verfahren
und eine Meßeinrichtung zur kolorimetrischen Bestimmung
insbesondere des Partialdrucks eines im Blut gelösten Gases zu schaffen, mit denen eine genaue und rasche Messung
insbesondere bei Anwendung in der Ergometrie, in der klinischen Routine, bei der Überwachung von Leistungssportlern
usw. möglich ist. Vor allem soll eine Kalibrierung der Meßeinrichtung entbehrlich werden, die bei den bisher bekannten Meßverfahren umständlich und zeitaufwendig ist.

Die erfindungsgemäße Lösung dieser Aufgabe ist hinsichtlich
des Verfahrens im Patentanspruch 1 angegeben.

Eine erfindungsgemäße Meßeinrichtung ist Gegenstand des
Patentanspruchs 4.

In vorteilhafter Ausgestaltung werden mit der Erfindung entsprechend der nachfolgenden Beschreibung zwei hinsichtlich
ihrer Meßgenauigkeit, aber auch ihres Aufwandes unterschiedliche Durchführungs- bzw. Ausgestaltungsmöglichkeiten angeboten, deren wesentliche Elemente kurz zusammengefaßt in den
Patentansprüchen 2 bzw. 3 und 5 bzw. 6 angegeben sind.

Vorteilhafte Weiterbildungen des Erfindungsgedankens sind in
Unteransprüchen gekennzeichnet.

Grundlage der erfindungsgemäßen Lösung ist eine bichromatische Messung am den zu erfassenden Stoff aufnehmenden
opto-chemischen Wandler, also eine Messung bei zwei unterschiedlichen Frequenzwerten der Meßstrahlung. Wird als opto-
chemischer Wandler (als Indikator) eine organische Säure
(z.B. Phenolrot) verwendet, so ergeben sich dann günstige

- 4 -

Meßbedingungen, wenn es für die dissoziierten ($A^-$) und die undissoziierten Komponenten (HA) des Indikatorfarbstoffs entsprechend der Reaktion $Ha-H_2 \rightleftarrows H_3O^+ + A^-$ eine Wellenlänge im Transmissionsspektrum der Farblösung gibt, für welche die Reaktionspartner, nämlich ($A^-$) und (HA) im Extinktionskoeffizienten übereinstimmen (isobestischer Punkt). Als optische Referenz ist diese Transmissionsstelle innerhalb des Spektrums der Wellenlängen bzw. Frequenzen, für die eine Änderung der Transmission in Abhängigkeit von dem zu erfassenden Stoff besteht, dadurch ausgezeichnet, daß der numerische Transmissionswert lediglich von der Indikatorfarbstoffkonzentration und der Temperatur beeinflußt wird. Die Transmission des Farbstoffs ist für diesen im folgenden mit "$\lambda_0$" bezeichneten Wellenlängenwert invariant gegen den Anteil am zu erfassenden Stoff bzw. in anderen Worten invariant gegen den pH-Wert der Farbstofflösung.

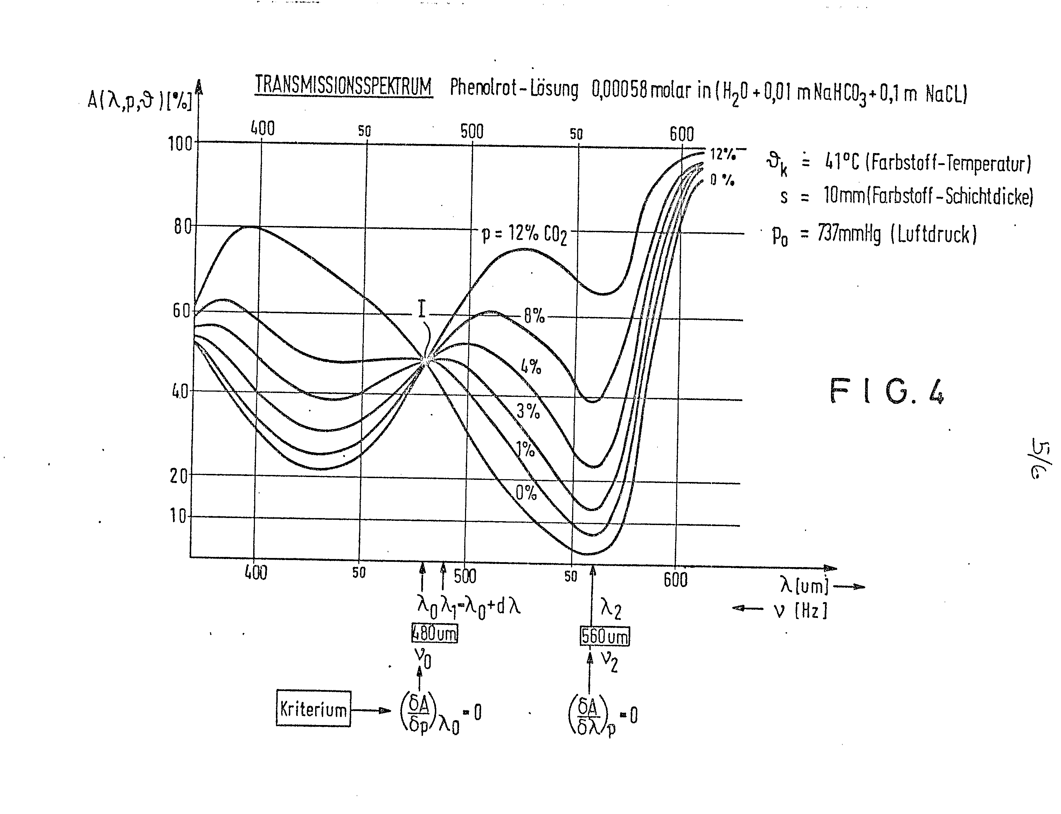

Die Erfindung nutzt den Verlauf des Kennlinienfeldes des Transmissionsspektrums eines solchen opto-chemischen Wandlers aus, wie es beispielhaft in der weiter unten näher erläuterten Fig. 4 der beigefügten Zeichnungen dargestellt ist. Dieses Transmissionsspektrum einer Indikator-Farblösung zeigt beim Einleiten von $CO_2$ für den Wellenlängenwert $\lambda_0$ den charakteristischen isosbestischen Punkt sowie bei einer Wellenlänge $\lambda_2 > \lambda_0$ ein Maximum der Abhängigkeit der Transmission vom Gehalt am zu erfassenden Stoff ($CO_2$).

Die Erfindung macht sich diesen charakteristischen Verlauf des Transmissionsspektrums zunutze und bietet zwei Verfahrensvarianten an:

1. Beim sogenannten "Differentialverfahren mit Referenz" beruht das Meßprinzip auf der Abhängigkeit des spektralen Differentialquotienten des Transmissionsgrades $\tau$ vom pH-Wert der Farbstofflösung.

- 5 -

Bezugswerte sind der Transmissionsgrad $\tau$ bei der Wellenlänge $\lambda_0$, für welche $\left(\dfrac{1}{\tau}\dfrac{\partial\tau}{\partial P}\right)_{\lambda_0} = 0$ und der Wellenlänge $\lambda_2$, für welche $\left(\dfrac{1}{\tau}\dfrac{\partial\tau}{\partial\lambda}\right)_P = 0$ gilt.

Gemessen werden die Empfänger-Signale $U(p)_{\lambda,T}$, wobei die spektralen Differentiale

$$B(p)_{\lambda,T} = \frac{1}{\tau}\frac{\partial\tau}{\partial\lambda}\,d\lambda$$

dem Dissoziationsgrad $\alpha$ des Farbstoffs und damit in erster Näherung dem $CO_2$-Partialdruck $pCO_2$ proportional sind.

Für die erfindungsgemäße kalibrierfreie Messung wird der Empfänger 11 dynamisch, also während der Messung, fortlaufend normiert, d.h. in anderen Worten, dem Transmissionsspektrum entsprechende Signalwerte werden unabhängig von Einflußgrößen wie Empfängerempfindlichkeit, Indikatorkonzentration und Strahlungsfluß unabhängig gemacht. Der die vom Indikatorfarbstoff remittierte Lichtstrahlung aufnehmende Empfänger wird für den Wellenlängenwert $\lambda_2$ alternierend mit Signalen aus einer optischen Referenzkammer (beispielsweise Partialdruck $pCO_2 = 0$) und der Meßkammer mit je der gleichen Farbstofflösung beaufschlagt, so daß bei Differenzmessung Fehler durch Änderung des Strahlungsflusses und der Empfängerempfindlichkeit eliminiert werden.

Bei einer vorteilhaften Ausgestaltungsform einer erfindungsgemäßen Meßeinrichtung ist eine Referenzkammer mit einem definierten Gehalt am zu erfassenden Stoff unmittelbar in der Meßsonde bzw. im transkutanen Aufnehmer enthalten.

2. Beim "Differentialverfahren ohne Referenz" beruht das Meßprinzip auf der Abhängigkeit des spektralen

Differentialquotienten $\left(\dfrac{1}{\tau}\dfrac{\partial \tau}{\partial \lambda}\right)_{\lambda_o,T}$ des Transmissionsgrades $\tau$ vom pH-Wert der Farbstofflösung im isobestischen Punkt, also bei Wellenlängenwert $\lambda_o$.

Gemessen werden hier bei den Wellenlängen $\lambda_o$ und $\lambda_1 = \lambda_o + d\lambda$, die Empfängersignale $U(p)$, wobei das spektrale Differential

$$B(p)_{\lambda_o,T} = \left(\frac{1}{\tau}\frac{\partial \tau}{\partial \lambda}d\lambda\right)_{\lambda_o,T} \sim \left(\frac{dU}{U}\right)_{\lambda_o,T}$$

bei der Temperatur T dem Dissoziationsgrad $\alpha$ des Farbstoffs und einem Störterm proportional ist, der die Abhängigkeit der spektralen Strahlungsflüsse bei $\lambda_o$ und $\lambda_1$ von der Strahlertemperatur und die unterschiedliche spektrale Empfindlichkeit des Empfängers berücksichtigt. Der Empfänger wird ebenfalls für $\lambda_o$ dynamisch normiert. Der den vom opto-chemischen Wandler remittierten Lichtanteil aufnehmende photoelektrische Empfänger zeigt für diese nahe benachbarten Wellenlängenwerte gleiche Empfindlichkeit, die gegebenenfalls durch geeignete Filter angepaßt werden kann.

Wie sich durch Berechnung und erste Messung ergibt, ist es für den Bereich $d\lambda \leqslant 5nm$ nicht erforderlich, eine optische Referenz für die Meßstelle $(\lambda_o + d\lambda)$ mitzuführen, da beispielsweise für thermische Strahler und lichtemittierende Dioden (LEDs) mit der angenähert spektralen Energieverteilung eines schwarzen Körpers das Verhältnis der spektralen Strahlungsflüsse $\emptyset(\lambda_o)$ und $\emptyset(\lambda_o+d\lambda)$ nur geringe Schwankungen in Abhängigkeit von der Farbtemperatur $T_f$ aufweist; z.B. bei $T_f = 3000$ K ergibt ein $T_f$ von $\pm 100$ K eine

Änderung des Verhältnisses der Strahlungsflüsse $\emptyset(\lambda_o)/\emptyset(\lambda_o + d\lambda)$ von nur etwa $\pm$ 1%.

Bei beiden Verfahrensvarianten kann die erforderliche Wellenlängenselektion mit Hilfe eines Echelette-Filters oder -Gitters, also eines Beugungsgitters mit treppenförmiger, spiegelnder Oberfläche vorgenommen werden. Die Blaze-Wellenlänge liegt vorzugsweise bei $\lambda_o$ und der mechanische Antrieb erfolgt durch einen im gewünschten Takt der Meßwerterfassung elektronisch gesteuerten Schrittmotor. Die mechanische Auflösung sollte zwischen 1,5 und 3 mrad oder $2^{11}$ bis $2^{12}$ Schritten pro Umdrehung liegen.

Als bevorzugtes Anwendungsgebiet steht die objektive kolorimetrische Bestimmung des pH-Werts einer pH-empfindlichen Farbstofflösung im Vordergrund, die über eine für das zu messende Stoffwechselprodukt (beispielsweise $CO_2$ oder $O_2$) durchlässige Membran an die Hautatmung eines Lebewesens gekoppelt ist und dadurch in Blut oder Gewebe befindliche Gase transkutan zu messen gestattet.

Das erfindungsgemäße Verfahren der bichromatischen Auswertung des Transmissions- oder Absorptionsspektrums einer Farblösung durch Differenzierung des Spektrums hinsichtlich Wellenlänge und pH-Empfindlichkeit bietet zum einen den Vorteil, daß sich damit ein einfach handhabbares Gerät ohne optische Referenz mit einem Fehler von höchstens 5% und langzeitstabilem Verhalten verwirklichen läßt.

Wird die bichromatische Auswertung des Transmissionsspektrums nach dem erwähnten Differentialverfahren mit optischer Referenz über einen optischen Vergleichskanal durchgeführt, so läßt sich bei wirksamer Unterdrückung von Störeinflüssen über große Applikationszeiten eine Genauigkeit von ungefähr

- 8 -

99% erreichen. Solche mit der Erfindung beseitigten Störeinflüsse sind vor allem Änderungen der spektralen Radianz
des optischen Senders, Änderungen der Temperatur und der
Konzentration der Farbstofflösung während der Meßzeit, Änderungen in den optischen Leitungen (z.B. Glasfasern) zwischen dem Sender und dem Meßwertaufnehmer bzw. zwischen
dem Meßwertaufnehmer und dem photoelektrischen Empfänger
sowie Änderungen der Empfängerempfindlichkeit durch Temperatur, Alterungseinflüsse und ähnliches.

Beide vorgestellten erfindungsgemäßen Verfahrensvarianten
sind nur durch Verwendung von Standardindikatoren und
Referenz-Farbwerten derselben sinnvoll anwendbar. Das elektronische Farbwert-Register ersetzt eine für visuelle,
kolorimetrische Meßverfahren übliche Farbskala, mit welcher
ein mit einer Untersuchungslösung reagierender Farbstoff
verglichen wird. Für die Berücksichtigung der Temperatur
der Farbwerte werden die Referenzwerte als Temperaturfunktionen mit parametrischer Differenz von etwa 0,1K gespeichert.
Die Selektion der der Temperatur T des Farbstoffs entsprechenden Farbwert-Spalte im Register wird mit einem Prozessrechner durchgeführt. Mit Hilfe dieser vorgespeicherten Bezugsfarbwerte entfällt auch jeder Aufwand für Linearisierung.

Die Erfindung und vorteilhafte Einzelheiten werden nachfolgende unter Bezug auf die Zeichnung in zwei beispielsweisen Ausführungsformen näher erläutert. Es zeigen:

Fig. 1  eine kolorimetrische Meßeinrichtung, der das er-
        findungsgemäße Meßprinzip nach dem Differential-
        verfahren ohne Referenz zugrunde liegt;
Fig. 2  eine kolorimetrische Meßeinrichtung erfindungsgemäßer Art nach dem Differentialverfahren mit
        Referenz;
Fig. 3  eine der Ausführungsform nach Fig. 2 ähnliche Meßeinrichtung nach dem Differentialverfahren
mit Referenz unter Verwendung nur eines die
remittierende Strahlung aufnehmenden Empfängers;

Fig. 3a in schematischer graphischer Darstellung
den zeitlichen Verlauf von Meßsignalen bei
der Meßanordnung nach Fig. 3;

Fig. 4 das Transmissionsspektrum einer Farbstofflösung;

Fig. 5 die Funktionsdarstellung der Anordnung nach

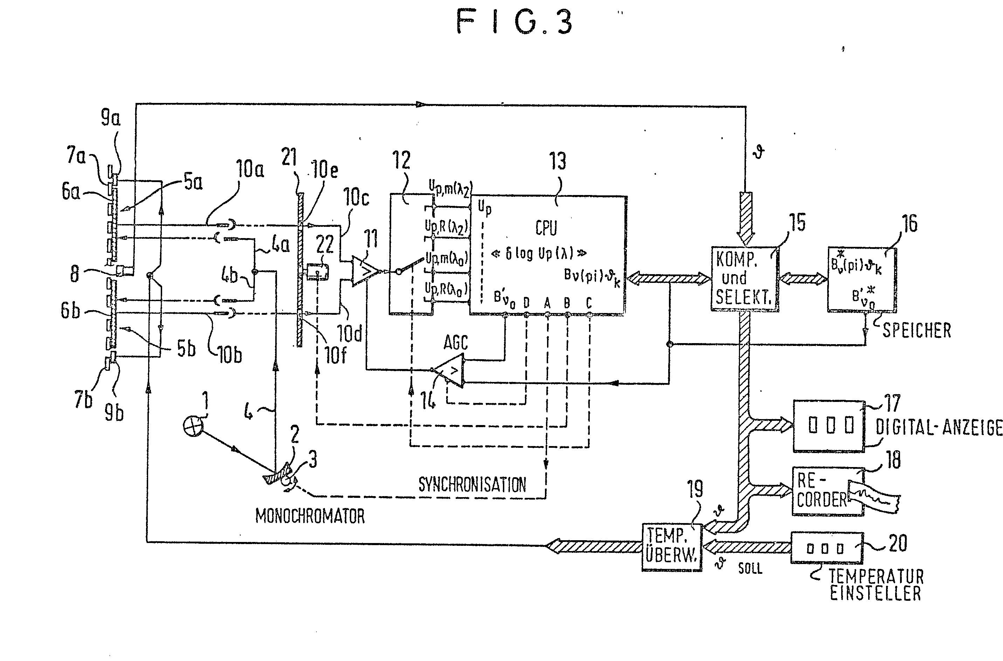

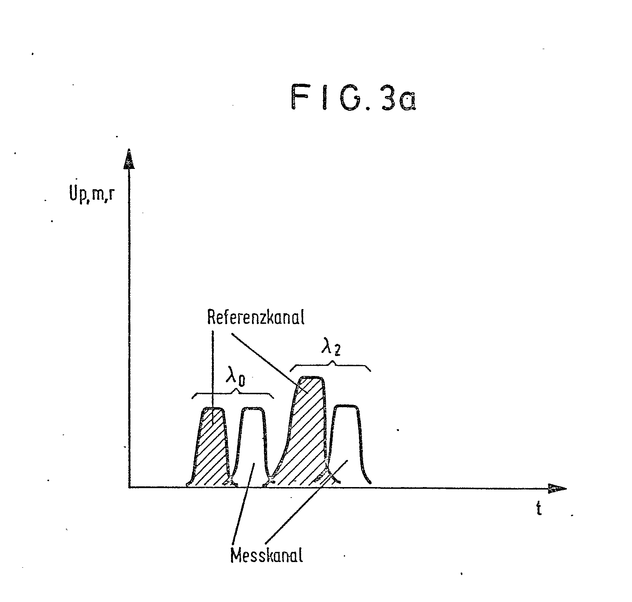

Fig. 3 zur Erläuterung der erfindungsgemäßen
dynamischen Normierung und der Auswahl von
vorgespeicherten Normspektren in Abhängigkeit
von der Temperatur einer in einem Sensor enthaltenen Farbstofflösung und

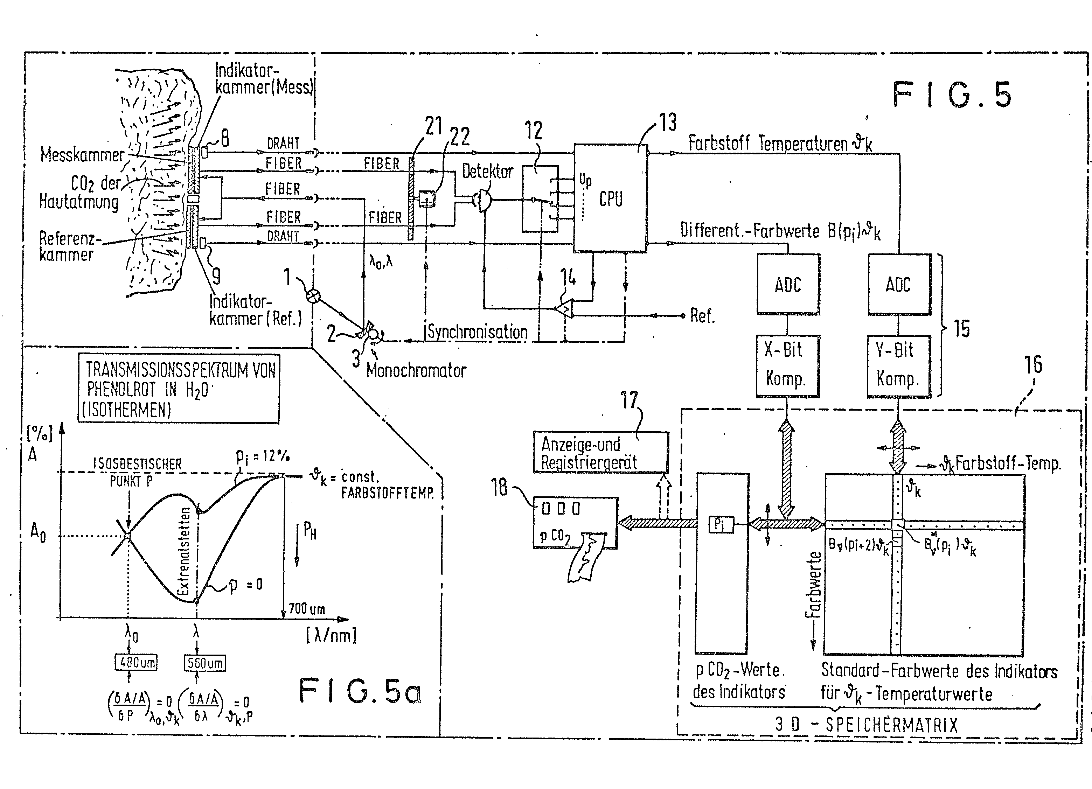

Fig. 5a einen Ausschnitt aus einem Transmissionsspektrum einer Farbstofflösung mit oberen und unteren Grenzkurven des pH-Werts zur Erläuterung
der Anordnung nach Fig. 5.

Einander entsprechende Baugruppen sind in den Figuren mit
den gleichen Bezugshinweisen gekennzeichnet.

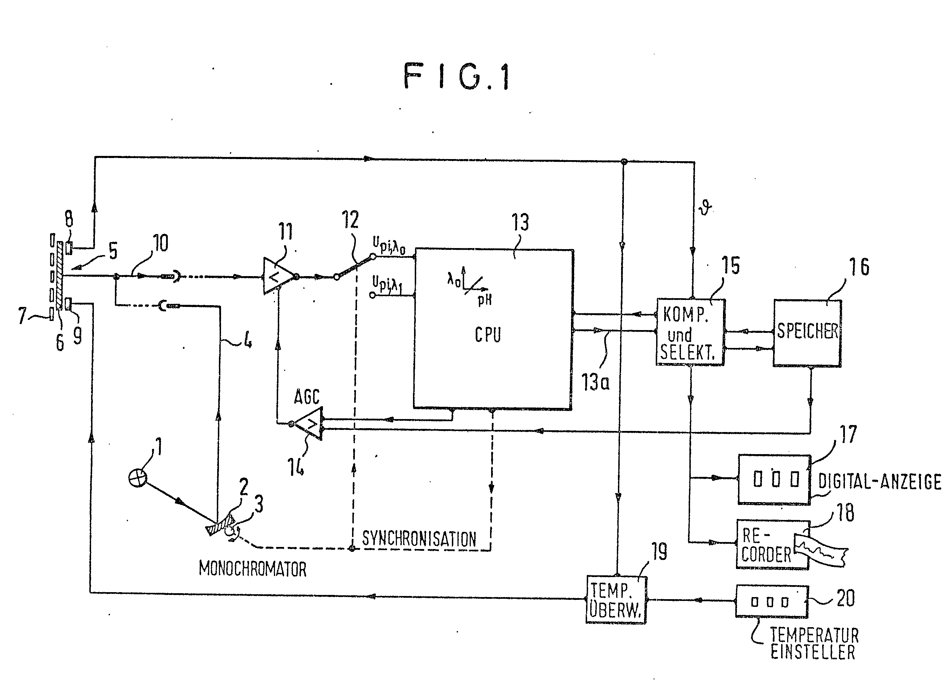

Bei der Anordnung einer erfindungsgemäßen kolorimetrischen
Meßeinrichtung nach Fig. 1 gelangt das Licht einer Strahlungsquelle 1 auf einen Monochromator, der im dargestellten
Beispiel aus einem Echelette-Filter 2 besteht, das durch
einen Schrittmotor 3 mit einer definierten, von einem Zentralprozessor 13 in weiter unten näher erläuterter Weise
synchronisierten Antriebsfrequenz schrittweise verstellt
wird. Die Blaze-Frequenz des Echelette-Filters 2 wird vorzugsweise zu etwa $\lambda_0$, also entsprechend der isobestischen
Wellenlänge einer verwendeten Indikator-Farbstofflösung gewählt.
Vom Monochromator 2, 3 gelangt - entsprechende Einstellung
des Monochromators vorausgesetzt - ein monochromatischer
Lichtstrahl der Wellenlänge $\lambda_0$ über eine faseroptische

- 10 -

Leitung 4 auf einen transkutanen Meßwertaufnehmer 5, der im folgenden als "Sensor" bezeichnet wird. Der nur im Prinzip dargestellte Sensor 5 umfaßt eine Meßkammer 6, deren an die Haut eines Probanden anzulegende Fläche mit einer Membran 7 bespannt ist, die für ein zu erfassendes Gas (beispielsweise $CO_2$) durchlässig, jedoch für andere Stoffwechselprodukte undurchlässig ist. Die vom monochromatischen Lichtstrahl zunächst getroffene rückseitige Fläche des Sensors 5 besteht aus einem transparenten Plättchen, welches auf der dem Inneren des Sensors 5 abgekehrten Fläche in genau definierter Weise aufgerauht ist. Die Definition der Aufrauhung ist in der Weise gegeben, daß für eine bestimmte Rautiefe eine über die optisch aktive Meßfläche vorliegende homogene Dichte von optisch wirksamen Streuzentren entsteht, deren Wirkung Voraussetzung für die Äquivalenz von Transmissions- und Remissionsspektrum des Farbstoffs ist. Auf diesem Sachverhalt beruht die beschriebene Messung der remittierten (diffus-reflektierten) Strahlung, wobei die Rauhtiefe die optisch wirksame Schichtdicke des Farbstoffindikators darstellt.

Eine andere Möglichkeit der Realisierung der oben genannten Äquivalenzbedienung ist die Verwendung eines Farbstoffes in Form eines hydrophilen Copolymerisats, dem submikroskopisch kleine Streusphären homogen beigemischt sind. Die Rauhtiefe dieses Plättchens bildet den wesentlichen Teil der eigentlichen Meßkammer 6, in welche eine Indikator-Farbstofflösung eingebracht ist, die beispielsweise für die Bestimmung des Partialdrucks von $CO_2$ (im folgenden "$pCO_2$") aus Phenolrot oder Bromthymolblau und allgemein aus einer organischen Säure bestehen kann, deren optische Absorptionseigenschaften sich bei gelöstem $CO_2$, also bei Veränderung des pH-Werts reversibel ändern. Der aus der Meßkammer 6 in Abhängigkeit vom momentanen durch den pH-Wert der Farbstofflösung bestimmten Extinktionsmodul re-

- 11 -

mittierende monochromatische Lichtanteil gelangt über eine Lichtleitung 10 auf einen opto-elektrischen Wandler 11 und von dort nach entsprechender Signalformung und Verstärkung über einen elektronischen Umschalter 12, dessen Funktion später noch erläutert wird, auf eine elektronische Auswerte bzw. Rechenschaltung 13, die nachfolgend als "Zentralprozessor" bezeichnet ist und Teil eines elektronischen Kleinrechners oder Mikroprozessors sein kann.

Wie bereits erwähnt, arbeitet die Anordnung der Meßeinrichtung nach Fig. 1 nach dem oben erläuterten Differentialprinzip, wobei ohne optische Referenz, also nur mit einer einzigen Meßkammer 6 gearbeitet wird. Diese Meßkammer enthält z.B. eine Indikatorlösung, die sich durch definierte Langzeitkonstanz ihrer chemo-optischen Eigenschaften auszeichnet. Als Indikatorspeicher und als Streuzentren für die remittierte Strahlung dient in diesem Fall, wie erwähnt, die Rauhtiefe einer geätzten Glasoberfläche, die der Membran 7 auf der vom monochromatischen Lichtstrahl zunächst getroffenen Fläche der Meßkammer 6 gegenüberliegt. Bei einem ersten Meßschritt gibt der Monochromator (vgl. auch das Transmissionsspektrum der Fig. 4) Licht der dem isosbestischen Punkt I entsprechenden Wellenlänge $\lambda_0$ ab.

Bei dieser Wellenlänge $\lambda_0$ verändert sich der Extinktionsmodul der Indikatorlösung in der Meßkammer 6 in Abhängigkeit vom pH-Wert nicht. Der opto-elektronische Wandler gibt jetzt ein elektrisches Meßsignal $U(p)\lambda_0$ an den Zentralprozessor 13 über den elektronischen Umschalter 12. Dieser Wert $U(p)\lambda_0$ wird in einem Kurzzeitspeicher festgehalten.

Im nächsten Meßschritt wird das Echelette-Gitter 2 durch den Schrittmotor 3 geringfügig verstellt, so daß jetzt monochromatisches Licht mit einer Wellenlänge

- 12 -

$\lambda_1 = (\lambda_0 \pm d\lambda)$ auf die Meßkammer 6 gelangt. $\lambda_1$ unterscheidet sich von $\lambda_0$ nur geringfügig (beispielsweise gilt $|\lambda_0 - \lambda_1| = d\lambda \leqq 5nm$. Bei diesem geringen Unterschied der beiden Meßwellenlängen zeigt der Empfänger bzw. optoelektrische Wandler 11 für beide Wellenlängen gleiche Empfindlichkeit; gegebenenfalls kann eine sehr genaue Anpassung der Empfindlichkeit durch ein vorgeschaltetes Filter erfolgen.

Das der Meß-Wellenlänge $\lambda_1$ entsprechende Meßsignal $U(p)_{\lambda_1}$ gelangt nach Umschaltung des elektronischen Schalters 12 auf einen anderen Eingang 12 des Zentralprozessors 13 und darin ebenfalls auf einen Zwischenspeicher. Im Zentralprozessor 13 wird sodann aus den gespeicherten Meßsignalwerten $U(p)_{\lambda_0}$ und $U(p)_{\lambda_1}$ das Differential des Transmissionsgrades der Lösung im isosbestischen Punkt I bei der Wellenlänge $\lambda_0$, also der Wert

$$B(p)_{\lambda_0,T} = \frac{1}{\tau}\left(\frac{\partial\tau}{\partial\lambda}d\lambda\right)_{\lambda_0,T}$$ gebildet. Wie schon erwähnt, sind die Farbwerte B dem Differential dlog $U(p)_{\lambda,T}$, d.h. dem Differential der logarithmierten Spannungswerte U des Empfängers äquivalent, die wiederum in eindeutiger Beziehung zum Extinktionsmodul des Farbstoffs, dem pH-Wert der Farbstofflösung und dem Störeinfluß stehen.

Die dem Speicher 16 zum Vergleich mit den akuten Farbwerten $B(p)_{\lambda,T}$ vorliegenden Standardwerte $B^*(p)_{\lambda,T}$ sind mit der methodisch gleichen Anordnung bestimmt worden wie die akuten Werte. Die dynamische Normierung der isosbestischen Farbwerte $B(p)_{\lambda_0,T}$ wird derart vorgenommen, daß die am Empfänger 11 für $\lambda_0$ auftretende Spannung $U(p)_{\lambda_0}$ vom Prozessor logarithmiert und an einen automatischen Regelverstärker 14 gegeben wird, dessen Referenzsignal $B^*\lambda_0$ von einem Speicher 16 vorgegeben ist. Auf diese Weise wird die Beeinflussung des photoelektrischen Signals insgesamt
berücksichtigt.

Als Ergebnis erhält man am Ausgang 13a des Zentralprozessors 13 einen bestimmten momentanen Farbwert $B(p)_{\lambda,T}$ des Indikators bei einer bestimmten Temperatur T des Farbstoffes für $p = pCO_2$. Dieser momentane Farbwert steht am einen Eingang einer Komparator- und Selektoreinheit 15 zur Verfügung, welche mit einem anderen Eingang mit dem Speicher 16 verbunden ist, dessen Aufbau im Zusammenhang mit der Fig. 5 weiter unten noch näher erläutert werden wird. Der Speicher 16 enthält also Standardfarbwerte $B^*(p)_{\lambda,T}$ des betreffenden Indikators für eine Reihe von Temperaturwerten T. Die momentan gültige Temperatur der Farbstofflösung in der Meßkammer 6 wird durch einen Temperaturfühler 8 erfaßt und der Komparator- und Selektoreinheit 15 mitgeteilt, so daß im Speicher 16 jeweils die dem betreffenden Temperaturwert zugeordnete Speicherebene für die Standardfarbwerte $B^*(p)_{\lambda,T}$ gewählt werden kann. Damit steht der Komparator- und Selektoreinheit 15 einmal der momentane akute Farbwert $B(p)_{\lambda,T}$ zur Verfügung. Durch Vergleich bestimmt die Komparator- und Selektoreinheit 15 den richtigen Standardfarbwert als Adresse für eine weitere Speicherebene, die zugeordnete Partialdruckwerte p bzw. $pCO_2$-Werte des Indikators enthält, die - gegebenenfalls nach erforderlicher Umsetzung - über eine Digitalanzeige 17 bzw. einen Recorder 18 als Punkt oder Abschnitt einer Meßkurve ausgegeben werden.

Die Temperatur der Indikatorlösung in der Meßkammer 6 des Sensors 5 wird durch eine Temperaturüberwachung 19 kontrolliert, welche eine Heizeinrichtung 9 im Sensor 5 speist. Eine gewünschte Sensortemperatur (z.B. im Bereich von 37 bis 45°C) läßt sich an einem Temperatureinstellelement 20 wählen.

Der Meßablauf mit Umstellung des Monochromators 2, 3 und entsprechender Umschaltung des elektronischen Schalters 12 wird vom Zentralprozessor 13 synchronisiert.

Der Verstärkungsgrad des optoelektrischen Wandlers 11 ist durch eine automatische Verstärkungsregelung 14 festgelegt, die einerseits vom Zentralprozessor 13 und andererseits von einem Standard-Speicherwert gespeist wird. Damit ist eine dynamische Normierung der Transmissionsmeßwerte auf den Transmissionswert für die Wellenlänge $\lambda_o$ möglich, so daß Einflußgrößen wie abweichende Indikatorkonzentration und Empfängerempfindlichkeit automatisch kompensiert werden.

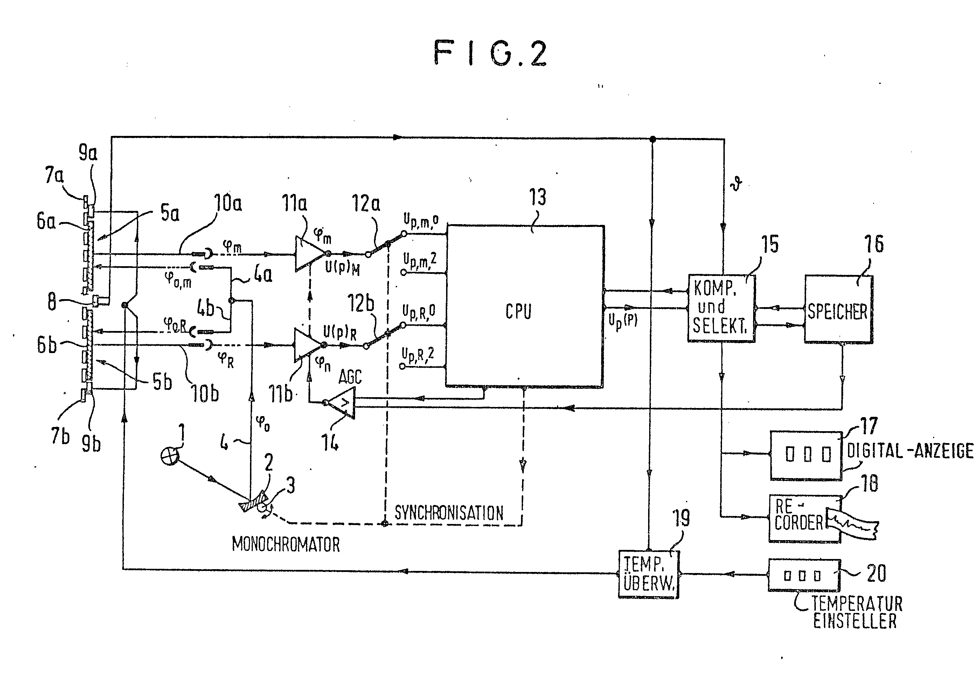

Die Meßanordnung nach Fig. 2 bzw. 3, die nach dem oben erläuterten Differentialverfahren mit Referenz arbeitet, unterscheidet sich hinsichtlich ihres äußeren Aufbaus von der Anordnung nach Fig. 1 im wesentlichen dadurch, daß eine optische Referenz über einen Referenzkanal mitgeführt wird. Der monochromatische Lichtstrahl gelangt vom Monochromator mit Echelettefilter 2 und Schrittschaltmotor 3 über die Lichtleitung 4 mit Verzweigungen 4a und 4b zum einen auf den Meßsensor 5a mit einer Meßkammer 6a, die als Kontaktfläche zur Meßstelle auf der Haut eines Lebewesens mit einer für das zu messende Gas durchlässigen Membran 7a bedeckt ist. Der über die Lichtleitungsverzweigung 4b angelieferte monochromatische Lichtanteil beaufschlagt zum anderen eine Referenzkammer 6b eines Referenzsensors 5b, der im Prinzip genauso aufgebaut sein kann wie der Meßsensor 5a, jedoch mit dem Unterschied, daß die in seiner Meßkammer 6b eingeschlossene Indikatorlösung einen definierten pH-Wert aufweist, der beispielsweise dem Wert $pCO_2 = 0$ entspricht. Die die Referenzkammer 6b abdeckende Membran 7b kann für diesen Fall durch eine licht- und gasundurchlässige Membran ersetzt sein oder die Membran wird mit einem definierten Anteil des zu messenden Gases beaufschlagt.
Die Sensoren 5a und 5b können in der Praxis zu einem einzigen Sensor vereinigt sein, wobei die Referenzkammer 6b
die Meßkammer 6a beispielsweise konzentrisch umgibt.

Der von der Meßkammer 6a remittierte Lichtstrahl gelangt
bei der Ausführungsform nach Fig. 2 auf einen photooptischen Wandler 11a und in analoger Weise wie bei Fig. 1
auf den Zentralprozessor 13. Dazu parallel geführt gelangt
der von der Referenzkammer 6b remittierte monochromatische
Referenzlichtanteil auf einen photo-optischen Wandler 11b
und über einen synchron mit dem elektronischen Umschalter
12a für das Meßsignal $U(p)_M$ betätigten elektronischen Umschalter 12b als Referenzsignal $U(p)_R$ auf einen anderen
Eingang des Zentralprozesssors 13. Die opto-elektronischen
Wandler 11a, 11b sind hinsichtlich ihres Verstärkungsgrades und zur dynamischen Normierung des Meßsignals auf den
Extinktionswert des isosbestischen Punkts automatisch verstärkungsgeregelt, ebenfalls analog der Ausführungsform
nach Fig. 1.

Der große Vorteil dieser Lösung des Meßproblems ist die
Freiheit vom Zwange einer Kalibrierung des Gesamtsystems
z.B. für O und 100% des Meßbereichs, dadurch, daß jeweils
gemessene Farbwerte mit gespeicherten Standard-Farbwerten
des Indikators verglichen werden.

Das gleiche gilt für eine mechanisch aufwendigere Alternative zur Anordnung nach Fig. 2, die in Fig. 3 dargestellt
ist, und auf deren zusätzliche Vorteile nachfolgend eingegangen wird.

Anstelle von zwei Wandlern ist die Verwendung nur eines
optischen Empfängers (Wandlers) 11 vorgesehen, dem über
die Lichtleiter 10c, 10d, die von einem mechanischen

- 16 -

Chopper 21, der durch einen Schrittmotor 22 verstellt wird, alternierend unterbrochenen Strahlungsflüsse 10e und 10f zugeführt werden. Am Ausgang des Empfängers 11 stehen dadurch in zeitlicher Folge die in Fig. 3a skizzierten Signalformen an.

Ein vom Zentralprozessor 13 in seiner Phasenlage zur Phasenlage der Schrittmoren 3 und 22 gesteuerter Demultiplexer 12 überträgt die am Ausgang des Empfängers 11 anstehenden Signale auf die Empfänger des Zentralprozessors 13. Die Ausgänge A, B, C des Zentralprozessors 13 sind nach definiertem Modus synchronisiert. Der opto-elektronische Empfänger 11 ist auch in dieser Version hinsichtlich seines Verstärkungsgrades automatisch auf einen vorgegebenen Pegel geregelt und zwar derart, daß der Verstärkungsgrad des Empfängers 11 bei Beaufschlagung der Meßanordnung mit Strahlungsleistung der Wellenlänge $\lambda_0$ auf den vorgegebenen Sollwert geregelt, der für alle anderen Phasen beibehalten wird.

Im Unterschied zum Differentialprinzip ohne Referenz bei der Anordnung nach Fig. 1 wird mit den Anordnungen nach Fig. 2 und Fig. 3 eine weitestgehende Ausblendung von Störeinflüssen erreicht.

Es werden zu diesem Zweck mit der Anordnung nach Fig. 2 bzw. Fig. 3 Farbwert-Differentiale $\partial U(\lambda)_p$ bei den Frequenzen $\lambda_0, \lambda_2$ ermittelt, für welche bei der isosbestischen Wellenlänge und an den Extremalstellen von $\tau$, für die die Ableitung von $\tau$ nach $\lambda$ bei beliebigen $pCO_2$-Werten verschwindet, die Beziehungen gelten:

$$\left(\frac{\partial \log U(p)}{\partial p}\right)_{\lambda_0} = 0 \qquad \text{und} \qquad \left(\frac{\partial \log U(\lambda)}{\partial \lambda}\right)_{p,\lambda_2} = 0$$

Die Farbwert-Differentiale werden unter Zuhilfenahme der gemessenen Spannungswerte U(p) am Empfänger 11 vom Prozessor 13 errechnet und zwar für den Meß- und Referenzkanal bei Wellenlängen $\lambda_0$ und $\lambda_2$ vor und nach Applikation des Sensors auf der Haut des Probanden.

Es werden vom Prozessor u.a. folgende Rechenoperationen durchgeführt (die Indizes M und R bezeichnen jeweils den Meßkanal bzw. den Referenzkanal):

Vor Applikation des Sensors mit $p = p_0 \; \hat{=} \; 0,03 \; \% \; CO_2$ bei Atmosphärendruck:

$$\log U_M (p_O)_{\lambda_o, T} \; - \; \log U_R (p_O)_{\lambda_o, T}$$

$$\log U_M (p_O)_{\lambda_2, T} \; - \; \log U_R (p_O)_{\lambda_2, T}$$

Nach Applikation des Sensors mit $p = pCO_2$ des Probanden:

$$\log U_M (p)_{\lambda_2, T} \; - \; \log U_R (p_O)_{\lambda_o, T}$$

$$\log U_M (p)_{\lambda_2, T} \; - \; \log U_R (p_O)_{\lambda_2, T}$$

Der Abgleich der Anordnung unter Benutzung der Ergebnisse der vorstehend genannten Rechen-Operationen bezüglich Nullpunkt, Störunterdrückung, Empfindlichkeit für die Meßgröße erfolgt fortlaufend durch den Prozeßrechner 13.

Die Anordnung nach Fig. 3 bietet hierdurch zusätzlich den Vorteil der Unabhängigkeit gegenüber Änderungen der Empfinlichkeit des Empfängers 11.

Die Bestimmung des Partialdruckwertes des zu erfassenden

- 18 -

Gases (z.B. pCo$_2$) erfolgt wiederum durch Vergleichen der
durch Messung gewonnen Werte des beispielsweise der Hautatmung des Probanden ausgesetzten Indikators bei der laufend überwachten Haut- bzw. Indikatortemperatur T mit im
Speicher 16 tabellierten Standardwerten der Indikatorlösung und entsprechender Auswahl des gesuchten p, z.B pCO$_2$.

Die Fig. 4 zeigt das bereits erwähnte Transmissionsspektrum
für eine Indikator-Farbstofflösung mit dem isosbestischen
Punkt I bei der Wellenlänge $\lambda_o$, bei welcher der Extinktionsmodul unabhängig wird vom Anteil an dem quantitativ zu erfassenden Stoff, also pH-Wert-unabhängig wird. Im isosbestischen Punkt ist der Extinktionsmodul lediglich abhängig von
der Indikatorkonzentration und von der Temperatur der Farbstofflösung.

Die Funktionsgruppenanordnung nach Fig. 5 dient in Verbindung mit dem Schaubild eines vereinfachten Transmissionsspektrums nach Fig. 5a in erster Linie zur Erläuterung
der Organisation des Speichers 16 und der Auswahl der
Standardwerte $B^*(p)_{\lambda,T}$ als Adresse für die gespeicherten
p-Werte (beispielsweise pCP$_2$-Werte) des Indikators.

Der Speicher 16 muß so organisiert sein, daß er zum einen
die Standard-Farbwerttabellen für einen bestimmten Indikator in Abhängigkeit von variablen Temperaturwerten aufnehmen kann und zum anderen die zugeordneten p-Werte der Indikator-Farbstofflösung enthält. Für eine Präzisionsmessung
ist es wünschenswert, daß die Temperatur-Auflösungsschritte
für die Speicherung um einen nennenswerten Faktor, beispielsweise 10 besser sind als die praktisch zu verwirklichende
Auflösung der Meßwerte. Dies bedeutet in der Praxis für
einen beispielshalber angenommenen Temperaturbereich von
35 bis 45°C und einer Unterteilung der Temperaturschritte im
Speicher auf 1/10°C, daß 100 Standard-Farbwerttabellen gespeichert sein müssen, deren Anzahl m von Farbwerten
$B^*(p)_{\lambda,T}$ (vgl. Fig. 5a) wiederum abhängig von der erwünschten Auflösung der pH-Wertangabe, beispielsweise der pCO$_2$-

- 19 -

Werte, die ausgegeben werden sollen, bezogen auf einen praktisch vorkommenden Konzentrationsbereich von beispielsweise 0% $CO_2$ bis 12% $CO_2$ (vgl. Fig. 4 und Kurven p = 0 und p = $p_{max}$ = 12% in Fig. 5a).

Zunächst wird entsprechend der durch den Temperaturfühler 8 mitgeteilten Temperatur T nach Analog/Digital-Umsetzung über den mit Y-Bit-Komp. bezeichneten Teil der Komparatorselektoreinheit 15, die für diesen Temperaturwert maßgebliche Standard-Farbwerttabelle festgelegt. Sodann bestimmt der mit X-Bit-Komp. bezeichnete Teil der Komparator- und Selektoreinheit 15 durch Vergleich des vom Zentralprozessor 13 angelieferten aktuellen Farbwerts $B(p)_{\lambda,T}$ mit einem entsprechenden Wert $B^*(p)_{\lambda,T}$ sozusagen eine "Adresse" für den Teil des Speichers 16, der die p-Werte, also die pH- bzw. Stoffkonzentrationswerte des betreffenden Indikators angibt. Der durch den Vergleich festgelegten Adresse zugeordnete p-Wert wird - gegebenenfalls nach Digital-, BCD- oder Digital/Analog-Umsetzung - an die Digitalanzeige 17 bzw. an das Aufzeichnungsgerät 18 abgegeben.

Verallgemeinert läßt sich sagen, daß der elektronische Speicher 16 für n Temperaturwerte n·m Normalfarbwerte der für die Messung verwendeten Indikator-Farbstofflösung enthält. Die Selektion der zum Vergleich mit den Meßwerten benötigten Farbwerte wird durch die Meßkammertemperatur T bewirkt. Die empirisch ermittelten und gespeicherten Normal-Farbwerte der Farbstofflösung ermöglichen eine direkte Bestimmung der Meßgröße pH (des p-Werts) ohne Kenntnis der Transferfunktion der pH-optischen Daten der Farbstofflösung, die für beide Farbwerte $B(p)_{\lambda,T}$ und $B(p)_{\lambda,T}$ identisch ist.

Durch die erfindungsgemäße bichromatische Meßmethode nach dem dargestellten Differentialprinzip mit Speicherung von

- 20 -

Normal-Farbwerten des verwendeten Farbstoffindikators für
verschiedene Temperaturwerte entfällt jeder Aufwand für
eine Linearisierung der Meßwertergebnisse, für Korrekturmaßnahmen, etwa zur Temperaturkompensation der opto-elektronischen Transferstrecken oder der Auswerteorgane. Das Verfahren läßt sich rasch und sicher auch von weniger geschultem Personal durchführen.

PATENTANSPRÜCHE


1. Verfahren zur kolorimetrischen Bestimmung der Konzentration eines chemischen Stoffs, insbesondere des Partialdrucks eines im Blut gelösten Gases, bei dem

- in einer abgeschlossenen Meßkammer ein opto-chemischer Wandler verwendet wird, dessen optische Eigenschaften sich entsprechend dem Gehalt an dem zu erfassenden chemischen Stoff reversibel ändern,

- der opto-chemische Wandler mit elektromagnetischer
Strahlung beaufschlagt wird, die Frequenzanteile
enthält, die von den optischen Eigenschaften des
opto-chemischen Wandlers in Abhängigkeit vom Gehalt
des zu erfassenden Stoffs unterschiedlich beeinflußt
werden,

- die vom opto-chemischen Wandler remittierte Strahlung photoelektrisch in ein elektrisches Meßsignal umgesetzt wird entsprechend der durch den Gehalt am zu erfassenden chemischen Stoff bestimmten
Intensität in dem zur Messung herangezogenen Spektralbereich, und bei dem

- das Meßsignal anschließend angezeigt und/oder registriert wird,

g e k e n n z e i c h n e t   d u r c h   folgende Verfahrensschritte:


- Es wird ein opto-chemischer Wandler mit Langzeitkonstanz seiner chemo-optischen Eigenschaften und
mindestens einem isosbestischen Punkt im Verlauf
seiner Wandlerkennlinie verwendet, bei dem für
einen bestimmten Wellenlängenwert $\lambda_0$ der Extinktionsmodul der Lösung invariant ist gegen die Konzentration des zu erfassenden Stoffs;

- es wird elektromagnetische Strahlung mindestens
zweier diskreter Wellenlängen $\lambda_0$ und $\lambda_{1,2}$ erzeugt
und auf den opto-chemischen Wandler gerichtet,

- 22 -

wobei $\lambda_0$ der isosbestische Wellenlängenwert für
den opto-chemischen Wandler ist, während der Wellenlängenwert $\lambda_{1,2}$ in einem Spektralbereich gewählt wird,
in welchem eine Abhängigkeit des Extinktionsmoduls
des opto-chemischen Wandlers vom Gehalt an dem zu
bestimmenden Stoff besteht;

- das elektrische Meßsignal wird einer elektronischen
Auswertung zugeführt, bei welcher das Meßsignal nach
einer vorgegebenen Transferfunktion in ein Farbwertsignal $B(p)_{\lambda,T}$ umgesetzt wird;

- es werden für diskrete Werte mindestens eines die
optischen Eigenschaften des opto-chemischen Wandlers
beeinflussenden Parameters eine Mehrzahl von auf
eine spezifische Eigenschaft des opto-chemischen
Wandlers bezogenen Standardwerte des Farbwertsignals und zugeordnete Stoffkonzentrationswerte gespeichert;

- das über die elektronische Auswertung aufgrund der
Messung erhaltene Farbwertsignal wird mit den zum
jeweiligen Meßzeitpunkt in Abhängigkeit vom maßgeblichen Parameterwert gültigen Speicherwerten des
Standard-Farbwertsignals verglichen;

- ein gespeicherter, dem Farbwertsignal zugeordneter
Stoffkonzentrationswert wird entsprechend dem Ergebnis des Vergleichs zur Anzeige und/oder Registrierung gebracht.

2. Verfahren nach Anspruch 1,
g e k e n n z e i c h n e t   d u r c h  folgende Verfahrensschritte:

- der Abstand $d\lambda$ der beiden Wellenlängen $\lambda_0$ und $\lambda_1$
der elektromagnetischen Strahlung wird auf einen
so kleinen Wert eingestellt, daß Abweichungen in
der Spektralempfindlichkeit eines photoelektrischen
Wandlerelements und anderer Einflußgrößen im remittierten Lichtweg vom opto-chemischen Wandler zur
photoelektrischen Umwandlung vernachlässigbar sind;

- es wird die Differenz der logarithmierten Meßsignale bei beiden Wellenlängen $\lambda_0$ und $\lambda_1$ gebildet;

- für eine Mehrzahl von Temperaturwerten des opto-
chemischen Wandlers wird eine empirisch ermittelte
Wertetabelle von aus vorgegebenen, für $\lambda_0$ und $\lambda_1$
erzeugten Differentialen gebildeten Farbwertsignalwerten gespeichert;

- entsprechend der jeweils vorherrschenden Temperatur
des opto-chemischen Wandlers wird die einem bestimmten momentanen Temperaturwert zugeordnete Wertetabelle der gespeicherten Farbwertsignale ausgewählt
und gegen den ermittelten angelieferten Ist-Wert
des Farbwertsignals verglichen;

- ein aus dem Vergleich resulierender Farbwert-Signalwert bestimmt die Auswahl eines zugeordneten gespeicherten Stoffkonzentrationswerts.

3. Verfahren nach Anspruch 1,
g e k e n n z e i c h n e t   d u r c h   folgende Verfahrensschritte:

- der gleiche Indikator wird als opto-chemischer Wandler in zwei gegeneinander abgedichtete Kammern eingebracht, von denen die eine als Meßkammer für den
zu erfassenden Stoff und die andere als Referenzkammer dient, in der die Konzentration des zu erfassenden Stoffs auf konstantem Wert gehalten wird;

- beide Kammern werden mit der gleichen elektromagnetischen Strahlung beider Wellenlängen $\lambda_0$ und $\lambda_2$
aufeinanderfolgend bestrahlt, wobei die Wellenlänge
$\lambda_2$ in einem Spektralbereich gewählt wird, für welche mindestens angenähert extremale Abhängigkeit
mindestens einer für die Messung verwendeten opti-

tischen Eigenschaft des Indikators von der Wellenlänge besteht;

- die von jeder Meßkammer remittierte Meß- bzw. Referenzstrahlung wird einer photoelektrischen Wandlung zugeführt;

- es wird die Differenz des von der Meßkammer und des
von der Referenzkammer erhaltenen logarithmierten
Meßsignals für beide Wellenlängenwerte $\lambda_0$ und $\lambda_2$
gebildet;

- für eine Mehrzahl von Werten der Temperatur T des Indikators wird eine Wertetabelle aus empirisch ermittelten Farbwertdifferenzen gespeichert, die aus
den Werten bei $\lambda_0$ und $\lambda_2$ gebildet sind;

- entsprechend der laufend gemessenen Temperatur des
Indikators wird eine dem jeweiligen Temperaturwert
zugeordnete Wertetabelle der gespeicherten Farbwerte ausgewählt;

- der Ist-Wert des durch Messung erhaltenen Farbwertsignals wird mit den Farbwertsignalen aus der ausgewählten Wertetabelle verglichen und das Vergleichsergebnis wird zur Auswahl eines zugeordneten gespeicherten Stoffkonzentrationswerts herangezogen.


4. Meßeinrichtung zur kolorimetrischen Bestimmung der Konzentration eines chemischen Stoffs, insbesondere des Partialdrucks eines im Blut gelösten Gases, mit

- einem Meßwertaufnehmer, der einen opto-chemischen
Wandler enthält, dessen optische Eigenschaften sich
entsprechend dem Gehalt an dem zu erfassenden chemischen Stoff reversibel ändern,

- einer Vorrichtung zur Beleuchtung des opto-chemischen
Wandlers mit elektromagnetischer Strahlung, die Wellenlängen enthält, an denen optische Eigenschaften
des opto-chemischen Wandlers in Abhängigkeit vom Gehalt des zu erfassenden Stoffs unterschiedlich beeinflußt werden,

- einem photoelektrischen Wandler zur Umsetzung der vom opto-chemischen Wandler auf den photoelektrischen Wandler geleiteten elektromagnetischen Strahlung in ein elektrisches Meßsignal entsprechend der durch den Gehalt am zu erfassenden chemischen Stoff bestimmten Intensität in dem zur Messung herangezogenen Spektralbereich, und mit

- einer Anzeige- und/oder Registriervorrichtung für das Meßsignal

g e k e n n z e i c h n e t  d u r c h  folgende Merkmale:

- Eine Komparator- und Selektoreinheit (15) vergleicht das über die Auswerteschaltung aufgrund der Messung erhaltene Farbwertsignal mit den, in Abhängigkeit von dem (den) zum jeweiligen Meßzeitpunkt maßgeblichen Parameterwert(en) gültigen Speicherwerten des Farbwertsignals und wählt einen zugeordneten Stoffkonzentrationswert aus, der - gegebenenfalls nach Umsetzung - der Anzeige- und/oder Registriervorrichtung (17, 19) zugeführt wird.

5. Meßeinrichtung nach Anspruch 4, g e k e n n z e i c h n e t  d u r c h  folgende Merkmale:

- der Abstand $d\lambda$ der beiden Wellenlängen $\lambda_0$ und $\lambda_1$ der durch die Beleuchtungsvorrichtung (1, 2, 3) abgegebenen elektromagnetischen Strahlung ist auf einen so kleinen Wert einstellbar, daß Abweichungen in der spektralen Empfindlichkeit des photoelektrischen Wandlers (11) und anderer Einflußgrößen im remittierten Lichtweg (10) vom opto-chemischen Wandler (6) zum photoelektrischen Wandler (11) vernachlässigbar sind;

- die elektronische Auswerteschaltung (13) enthält

- 26 -

eine Logarithmier- und Differenzstufe, die die
Differenz der logarithmierten Meßsignle der beiden
Wellenlängen $\lambda_0$ und $\lambda_1$, $\log U(p)_{\lambda_0, T}$ - $\log U(p)_{\lambda_1, T}$
bildet;

- der Speicher (16) enthält für jeden einer Mehrzahl
  von Temperaturwerten des opto-chemischen Wandlers
  eine Wertetabelle von aus vorgegebenen Differentialen

  bei $\lambda_0$, $\left( \dfrac{1}{\tau} \dfrac{\partial \tau}{\partial \lambda} d\lambda \right)_{\lambda_0, T}$ ,gebildeten Farbwertsignalwerten.

6. Meßeinrichtung nach Anspruch 4,
   g e k e n n z e i c h n e t   d u r c h  folgende Merkmale:
   - der Meßwertaufnehmer (5) umfaßt zwei den gleichen Indikator als opto-chemischen Wandler enthaltende gegeneinander abgedichtete Kammern (6a, 6b), von denen
     die eine als Meßkammer (6a) für den zu erfassenden
     Stoff und die andere als Referenzkammer (6b) dient,
     in der die Konzentration des zu erfassenden Stoffs
     auf einem konstanten Wert gehalten wird;
   - beide Kammern werden durch die Beleuchtungsvorrichtung (1, 2, 3) mit der gleichen elektromagnetischen
     Strahlung beider Wellenlängenwerte $\lambda_0$ und $\lambda_2$ auf-
     einanderfolgend bestrahlt und die Wellenlänge $\lambda_2$
     ist in einem Spektralbereich gewählt, für welchen
     mindestens angenähert extremale Abhängigkeit mindestens einer für die Messung verwendeten optischen
     Eigenschaft des Indikators von der Wellenlänge
     besteht;
   - die von jeder Kammer (6a, 6b) remittierte Meß- bzw.
     Referenzstrahlung gelangt auf einen photoelektrischen
     Wandler (11; 11a, 11b), dessen Ausgangssignal der
     elektronischen Auswerteschaltung (13) zugeführt wird;
   - die elektronische Auswerteschaltung (13) enthält
     eine Logarithmier- und Differenzstufe, welche die Differenz der von der Meßkammer und der von der Referenzkammer erhaltenen logarithmierten Meßsignale log $U(p)_{\lambda_o,T}$ $-$log $U(p)_{\lambda_2,T}$ für beide Wellenlängen $\lambda_o$ und $\lambda_2$ bildet;

- der Speicher (16) enthält für jeden einer Mehrzahl von Temperaturwerten T des Indikators eine Wertetabelle von aus vorgegebenen, für $\lambda_o$ und $\lambda_2$ erzeugten Differenzen gebildeten Farbwertsignalwerten;

- die Komparator- und Selektoreinheit (15) ist auf die jeweils gemessene Temperatur des Indikators einstellbar und wählt zunächst die einem jeweiligen Temperaturwert zugeordnete Wertetabelle der Farbwertsignale im Speicher (16) und vergleicht anschliessend den von der Auswerteschaltung (13) angelieferten Ist-Wert des Farbwertsignals mit den Farbwertsignalen der ausgewählten Wertetabelle und bestimmt bei Gleichheit den zugeordneten gespeicherten Stoffkonzentrationswert (p).

7. Meßeinrichtung nach einem der vorstehenden Ansprüche 4 bis 6,
d a d u r c h   g e k e n n z e i c h n e t , daß der opto-chemische Wandler ein flüssiger oder feinkörniger Indikator mit Langzeitkonstanz seiner opto-chemischen Eigenschaften ist.

8. Meßeinrichtung nach Anspruch 7,
d a d u r c h   g e k e n n z e i c h n e t , daß der opto-chemische Wandler in Form einer Indikator-Lösung in die definiert geätzte Rauhtiefe eines transparenten Plättchens eingebracht ist, die Streuzentren für die remittierte Strahlung bildet, die von dem Sensor über die Faseroptik an den Detektorabgegeben wird.

9. Meßeinrichtung nach Anspruch 4 oder 6,
d a d u r c h   g e k e n n z e i c h n e t , daß die
elektronische Auswerteschaltung (13) einen Prozessrechner enthält, der die vom photoelektrischen Wandler gelieferten Meßsignale entsprechend der vorgegebenen Transferfunktion so verarbeitet, daß ein Vergleich mit den
Standardwerten des opto-chemischen Wandlers im Speicher
ermöglicht ist.

10. Meßeinrichtung nach Anspruch 5,
d a d u r c h   g e k e n n z e i c h n e t , daß der
Meßwertaufnehmer (6) ein transkutaner Sensor ist, dessen
Meßkammer eine vorwiegend in der Rauhtiefe einer definiert oberflächengeätzten transparenten Fläche eingebrachte pH-Wert-empfindliche Farbstofflösung als Indikator enthält, die auf der der transparenten Fläche gegenüberliegenden Seite über eine für den zu erfassenden Stoff
durchlässige Membran an die Hautatmung eines Lebewesens
anschließbar ist.

11. Meßeinrichtung nach Anspruch 9,
d a d u r c h   g e k e n n z e i c h n e t , daß die
von der Meßkammer (6a) bzw. von der Referenzkammer (6b)
remittierte Strahlung über einen vom Prozeßrechner (13)
im Takte der Umschaltung von Referenz- und Meßsignalerfassung geschalteten Zerhacker auf ein und denselben
photoelektrischen Wandler (11) gelangt.

12. Meßeinrichtung nach Anspruch 5 oder 6,
d a d u r c h   g e k e n n z e i c h n e t , daß der
(die) photoelektrische(n) Wandler (11; 11a, 11b) über
eine automatische Verstärkungsregelschaltung (14) auf
einen bei dem Wellenlängenwert $\lambda_0$ vorgegebenen Standardwert des Meßsignals dynamisch normierbar ist (sind), wobei die Eingangsgrößen der Verstärkungsregelschaltung zum einen vom Prozeßrechner (13), der den Ist-Wert
des opto-elektrischen Signals ermittelt und zum anderen als Standardwert (Normwert) vom Speicher (16)
geliefert werden.

13. Meßeinrichtung nach Anspruch 6,
d a d u r c h   g e k e n n z e i c h n e t , daß der
Ausgang jedes opto-elektrischen Wandlers (11; 11a, 11)
unter Steuerung durch den Prozeßrechner (13) auf zwei
unterschiedliche jeweils einem opto-elektrischen Wandler zugeordnete Eingänge des Prozeßrechners schaltbar
ist, von denen der eine Eingang bei Messung mit dem
Wellenlängenwert $\lambda_0$ und der andere Eingang bei Messung
mit dem Wellenlängenwert $\lambda_2$ mit dem zugeordneten opto-
elektrischen Wandler verbunden ist.

14. Meßeinrichtung nach einem der vorstehenden Ansprüche 4
bis 13,
d a d u r c h   g e k e n n z e i c h n e t , daß als
die Meßstrahlung frequenzselektierende Einrichtung in
der Beleuchungsvorrichtung ein Gittermonochromator (2)
vorgesehen ist, dessen optische Orientierung seiner
Hauptnormalen zur von einer Strahlungsquelle aus einfallenden weißen Strahlung durch die vom Prozeßrechner (13)
gesteuerte Drehbewegung eines mit dem Gitter verbundenen
Schrittmotors (3) erfolgt.

15. Meßeinrichtung nach Anspruch 6,
d a d u r c h   g e k e n n z e i c h n e t , daß die
Meßkammer (6a) und die Referenzkammer (6b) gemeinsam
über Lichtleitungen (4a, 4b) an den Strahlausgang der
Beleuchtungseinrichtung (1, 2, 3) so angeschlossen sind,
daß beide Kammern gleichzeitig mit der elektromagnetischen
Strahlung zunächst der einen und dann der anderen Wellenlänge $\lambda_0$ und $\lambda_2$ beleuchtbar sind, und daß die Meßkammer

- 30 -

und die Referenzkammer über getrennte Lichtleitungen
(10a, 10b) mit dem jeweils zugeordneten opto-elektrischen Wandler verbunden sind.

16. Meßeinrichtung nach einem der vorstehenden Ansprüche 4
bis 15,
d a d u r c h   g e k e n n z e i c h n e t , daß an
den Meßwertaufnehmern ein die Temperatur des Indikators
erfassender Temperaturfühler (8) angeschlossen ist,
dessen Ausgangssignal der Komparator- und Selektoreinheit (15) zugeführt wird.

17. Meßeinrichtung nach einem der vorstehenden Ansprüche 4
bis 16,
d a d u r c h   g e k e n n z e i c h n e t , daß der
Verstärkungsgrad des opto-elektrischen Wandlers fortlaufend für den Wellenlängenwert $\lambda_0$ automatisch geregelt
ist.

18. Meßeinrichtung nach Anspruch 17,
d a d u r c h   g e k e n n z e i c h n e t , daß die
Verstärkerregelung für den opto-elektrischen Wandler
unter Steuerung durch den Zentralprozessor (13) so erfolgt, daß das auf den Zentralprozessor (13) gelangende
Meßsignal dynamisch auf den Standardwert bei der isosbestischen Wellenlänge $\lambda_0$ normiert wird.

19. Meßeinrichtung nach einem der vorstehenden Ansprüche
4 bis 18,
d a d u r c h   g e k e n n z e i c h n e t , daß der
Meßwertaufnehmer zur transkutanen Bestimmung des pH-
Werts bzw. des Partialdrucks von Kohlendioxid im Blut
eingerichtet ist.

20. Meßeinrichtung nach Anspruch 19,

d a d u r c h   g e k e n n z e i c h n e t , daß als
Indikator die Farbstofflösung Phenolrot dient, die in
der Meßkammer (6) und gegebenenfalls in der Referenzkammer (6b) in Form einer Lösung in der Rauhtiefe eines definiert tiefengeätzten Glassubstrats oder in Form
eines in der Lösung getränkten Substrats wie Filz, Papier,
Schwamm oder poröser Palettenform enthalten ist.

21. Meßeinrichtung nach Anspruch 6,

d a d u r c h   g e k e n n z e i c h n e t , daß die
Meßkammer (6) und die Referenzkammer (6b) konzentrisch
im Meßwertaufnehmer angeordnet sind, die Referenzkammer
(6b) vorzugsweise innerhalb der ringförmigen Meßkammer
(6a).

22. Meßeinrichtung nach einem der vorstehenden Ansprüche 4
bis 21,

g e k e n n z e i c h n e t   d u r c h   eine im Meßwertaufnehmer (5) eingebaute elektrische Heizeinrichtung,
deren Heizleistung durch einen vom Ausgangssignal des
Temperaturfühlers (8) beaufschlagten Temperaturregler
(19) entsprechend der Einstellung an einem Temperatureinstellglied (20) regelbar ist.

# FIG.1

# FIG.2

SYNCHRONISATION

MONOCHROMATOR

CPU

KOMP. und SELEKT.

SPEICHER

DIGITAL-ANZEIGE

RE-CORDER

TEMP. ÜBERW.

TEMPERATUR EINSTELLER

AGC

# FIG.3

# FIG.3a

Up,m,r

Referenzkanal

$\lambda_2$

$\lambda_0$

Messkanal

t

TRANSMISSIONSSPEKTRUM Phenolrot-Lösung 0,00058 molar in ($H_2O$ + 0,01 m $NaHCO_3$ + 0,1 m NaCL)

FIG. 4

$\vartheta_k \doteq 41°C$ (Farbstoff-Temperatur)

s = 10mm (Farbstoff-Schichtdicke)

$P_0$ = 737mmHg (Luftdruck)

p = 12% $CO_2$

Kriterium $\rightarrow$ $\left(\dfrac{\delta A}{\delta p}\right)_{\lambda_0} = 0$  $\left(\dfrac{\delta A}{\delta \lambda}\right)_p = 0$

$\lambda_0$ $\lambda_1 = \lambda_0 + d\lambda$  $\lambda_2$

480 um  560 um

$\nu_0$  $\nu_2$

$\lambda$ [um] $\rightarrow$

$\leftarrow$ $\nu$ [Hz]

FIG. 5

FIG. 5a